**Europäisches Patentamt**

**European Patent Office**

**Office européen des brevets**

(19)

(11) Veröffentlichungsnummer : **0 015 508**
**B1**

(12) **EUROPÄISCHE PATENTSCHRIFT**

(45) Veröffentlichungstag der Patentschrift :
30.09.81

(51) Int. Cl.³ : **C 12 Q   1/50, G 01 N 33/70**

(21) Anmeldenummer : **80100995.2**

(22) Anmeldetag : **28.02.80**

(54) **Verfahren und Reagenz zur Bestimmung der Creatinkinase-MB.**

(30) Priorität : **02.03.79 DE 2908053**

(43) Veröffentlichungstag der Anmeldung :
**17.09.80 (Patentblatt 80/19)**

(45) Bekanntmachung des Hinweises auf die Patenterteilung : **30.09.81 Patentblatt 81/39**

(84) Benannte Vertragsstaaten :
**AT BE CH DE FR GB IT LU NL SE**

(56) Entgegenhaltungen :
**DE - A - 2 548 962**
**FR - A - 2 330 010**
**GB - A - 1 521 986**
**GB - A - 2 026 156**

**CHEMICAL   ABSTRACTS,   Band   89,   nr. 17,
23. Oktober   1978,   Zusammenfassung   Nr. 142
374 m, Seite 229**

**CHEMICAL   ABSTRACTS,   Band   91,   nr. 11,
10. September 1979, Zusammenfassung, Nr. 86
090 t, Seite 311**

**CLINICA CHIMICA ACTA, Band 58, 1975, Seiten
223 bis 232**

(73) Patentinhaber : **BOEHRINGER MANNHEIM GMBH
Sandhofer Strasse 112-132 Postfach 31 01 20
D-6800 Mannheim 31-Waldhof (DE)**

(72) Erfinder : **Gruber, Wolfgang, Dr.
Am Oberanger 7
D-8132 Tutzing-Unterzeismering (DE)**
Erfinder : **Lenz, Helmut, Dr.
Waldschmidtstrasse 7
D-8132 Tutzing (DE)**
Erfinder : **Looser, Siegfried, Dr.
Ammerstrasse 27
D-8120 Weilheim (DE)**
Erfinder : **Linke, Hans-Ralf, Dr.
Andechser Strasse 7
D-8919 Raisting (DE)**

(74) Vertreter : **Weickmann, Heinrich, Dipl.-Ing Patentanwälte Dipl.Ing.H.Weickmann et al
Dipl.Phys.Dr.K.Fincke Dipl.Ing.F.A.Weickmann
Dipl.Chem.B.Huber, Dr.-Ing.H.Liska Möhlstrasse 22
D-8000 München 86 (DE)**

# Verfahren und Reagenz zur Bestimmung der Creatinkinase-MB

Die Erfindung betrifft ein Verfahren und ein Reagenz zur Bestimmung der Aktivität von Creatinphosphokinase MB, im folgenden CK-MB genannt, im Serum.

Im menschlichen Körper kommen zwei verschiedene Arten von Untereinheiten dieses Enzyms vor, die Untereinheiten M und B. Da das aktive Enzym aus jeweils zwei Untereinheiten zusammengesetzt ist und da die beiden Untereinheiten frei miteinander kombinieren können, sind drei Enzymtypen möglich : der Muskeltyp CK-MM, der Gehirntyp CK-BB und der Hybridtyp CK-MB, der hauptsächlich im Myokard vorkommt, beim Herzinfarkt in das Serum austritt und dann dort in erhöhter Konzentration zu finden ist. Die Aktivität dieses Hybrid-Isoenzyms kann neben der Gesamtaktivität der CK im Serum für die Diagnose des Herzinfarkts herangezogen werden.

Es ist bekannt, durch Zusatz von spezifischen Antikörpern gegen die Untereinheit M der CK den im Serum vorkommenden Muskeltyp des Isoenzyms CK-MM auszuschalten und dann nach einer der bekannten Methoden der CK-Bestimmung das noch verbleibende Hybridenzym CK-MB zu messen. Dabei wurden sowohl präzipitierende als auch hemmende Antikörper verwendet. Ein Nachteil bestand jedoch darin, daß hierbei auch das Hybridenzym CK-MB zu 80 % gehemmt wird (Clin. Chim. Acta, *58*, 223-232 (1975)). Es war zwar bereits bekannt, Antikörper zu gewinnen, welche den Muskeltyp CK-MM vollständig und den Gehirntyp CK-BB überhaupt nicht hemmen (Immunochemistry, Vol. 6, 681-687 (1969)). Obwohl bei derartigen Antikörpern die Untereinheit B im Hybridenzym CK-MB überhaupt nicht gehemmt wird und daher hiermit die Zuverlässigkeit der Bestimmungsmethode erhöht wird, weist auch dieses Verfahren noch erhebliche Nachteile auf. Es hat sich nämlich gezeigt, daß selbst bei Verwendung reinster Antigene (CK-MM) die für die Antikörperbildung verwendeten Tiere überwiegend ein Serum lieferten, welches das Hybridenzym CK-MB zu mehr als 55 %, zumeist zwischen 60 und 90 %, hemmten. Daher war es nötig, bei jedem einzelnen, für die Immunisierung verwendeten Tier zu untersuchen, ob die gebildeten Antikörper die CK-MB nur zu 50 % oder aber stärker hemmten. Abgesehen von dem hierdurch verursachten Aufwand führt dies dazu, daß der größte Teil der gewonnenen Seren praktisch nicht verwendet werden kann, da stets verschiedene Hemmungsgrade auftreten. Auch gepoolte Seren beseitigen diese Schwierigkeit nur teilweise. Hinzu kommt weiter, daß die CK-MB-Menge im Serum bei Herzinfarkt äußerst gering ist und bei Antikörpern, die wiederum einen erheblichen Teil davon ausschalten, die Zuverlässigkeit des Verfahrens stark vermindert wird.

Der Erfindung liegt die Aufgabe zugrunde, diese Nachteile zu beseitigen und ein Verfahren der angegebenen Art zu schaffen, welches es insbesondere möglich macht, auch Antikörper, welche die CK-MM vollständig, die CK-MB jedoch zu mehr als 55 % hemmen, so einsetzen zu können, daß die gesamte Aktivität der im Hybridenzym CK-MB enthaltenen Untereinheit B bestimmt werden kann.

Gelöst wird diese Aufgabe erfindungsgemäß durch ein Verfahren zur Bestimmung der Creatinkinase-MB im Serum durch immunologische Ausschaltung der Untereinheit M und Messung der Untereinheit B nach bekannten Methoden zur Bestimmung der Creatinkinase, welches dadurch gekennzeichnet ist, daß man zur Ausschaltung der Untereinheit M im Reaktionsansatz aus Antikörpern gegen die Untereinheit M durch proteolytische Spaltung unter reduzierenden Bedingungen gewonnene monovalente Fragmente zusetzt.

Es ist zwar bekannt, daß Enzyme nicht nur von den natürlichen Antikörpern, die der IgG-Fraktion bzw. den γ-Globulinen angehören und ein Molekulargewicht von etwa 130 000 bis 210 000 aufweisen, gehemmt werden, sondern daß auch durch proteolytische Spaltung dieser Antikörper Fragmente entstehen, die zwar die Präzipitationsfähigkeit verloren haben, da sie nur noch monovalent sind im Gegensatz zu den divalenten Antikörpern, daß jedoch die Hemmeigenschaften von monovalenten Fragmenten sich nur geringfügig von denen der divalenten Antikörper unterscheiden (Kontakte 3/78, 10-17). Sie werden als FAB-Fragmente bezeichnet (Biochem. J. *73*, 119-126 (1959) ; Immunochem. *4*, 369 (1967)).

Überraschenderweise wurde nun gefunden, daß dies im vorliegenden Falle nicht zutrifft, sondern aus Antikörpern, welche die CK-MM vollständig und die CK-MB zu mehr als 55 %, insbesondere zu 60 bis 90 %, hemmen, monovalente Fragmente (FAB-Fragmente) erhalten werden, welche die CK-MM immer noch vollständig, d.h. zu 99 bis 100 %, die CK-MB jedoch im Rahmen der Fehlergrenze nur zu 50 % hemmen. Hierdurch wird es möglich, die von geeigneten Versuchstieren, wie Schafen, Kaninchen, Hühnern und dergleichen, gewonnenen Antiseren für die Bestimmung der CK-MB einzusetzen, ohne daß vorher durch aufwendige analytische Methoden diejenigen Antiseren ausgesondert werden müssen, welche die CK-MB zu mehr als etwa 53 % hemmen. Vielmehr ist es nunmehr möglich, die Seren ohne vorherige Bestimmung ihrer Hemmeigenschaften proteolytisch unter reduzierenden Bedingungen zu spalten und mit den Spaltfragmenten dann die Untereinheit M der CK, gleichgültig ob im Muskelenzym CK-MM oder im Hybridenzym CK-MB vorhanden, spezifisch auszuschalten. Bevorzugt werden im Rahmen der Erfindung monovalente Fragmente verwendet, die aus Antikörpern gewonnen werden, welche das Hybridenzym CK-MB zu mehr als 55 %, besonders bevorzugt zu 60 bis 90 %, und das

Muskeltyp-Isoenzym CK-MM vollständig hemmen.

Gemäß einer weiteren bevorzugten Ausführungsform der Erfindung verwendet man solche monovalenten Fragmente, aus welchen restliche vollständige Antikörper und die kristallisierbaren Fragmente ($F_c$) abgetrennt sind. Die Abtrennung kann nach bekannten Methoden erfolgen, beispielsweise durch Gelchromatographie oder durch Dialyse gegen Salzlösung und Fällung mit Zinksulfat (Immunochem. *14*, 99 (1977)).

Die in bekannter Weise erhaltenen monovalenten Fragmente, die, wie erwähnt, vorzugsweise von Antikörpern und $F_c$-Fragmenten gereinigt werden, lassen sich als solche im Verfahren der Erfindung einsetzen. Bevorzugt werden jedoch monovalente Fragmente verwendet, deren SH-Gruppen vorher alkyliert wurden. Geeignet sind diejenigen Alkylierungsmittel, welche in wäßrigem Medium bei pH-Werten, welche die Tertiärstruktur von Proteinen nicht verändern, wirksam sind. Beispiele hierfür sind Jodacetat und Jodacetamid, wobei letzteres bevorzugt verwendet wird, da es gleichzeitig die zur Herstellung der Fragmente eingesetzten proteolytischen Enzyme zu inaktivieren vermag.

Bei der Herstellung der zur Gewinnung der erfindungsgemäß verwendeten FAB-Fragmente benötigten Antiseren kommen übliche Immunisierungsmethoden zur Anwendung, wie sie in der Literatur vielfach beschrieben sind. Hingewiesen wird beispielsweise auf « Methods of Immunology and Immunochemistry », Band 4, 313 ff (1976), insbesondere Seite 336. Generell kommt es bei der Immunisierung darauf an, möglichst reine Antigene zu verwenden und ausreichend lange zu immunisieren. Die allgemeinen Methoden werden beispielsweise beschrieben in « Immunologische Arbeitsmethoden », VEB Gustav Fischer Verlag Jena, 1976, 368-370, und « Microbiology », Verlag Harper & Row, New York, 1970, 458/459. Für die Immunisierung geeignete reine CK-MM-Isoenzyme (Antigene) lassen sich nach ABB *150*, 648-678 (1972) gewinnen, wobei zweckmäßig bei der Isolierung und Reinigung dem Puffer Dithiotreitol zur Stabilisierung zugesetzt wird.

Aus den Antiseren wird in üblicher Weise die IgG-Fraktion bzw. das Gammaglobulin gewonnen und anschließend unter reduzierenden Bedingungen mit einem proteolytischen Enzym gespalten, welches an der Hinge-Region angreift. Beispiele hierfür sind Papain und Pepsin. Die Spaltung ist beispielsweise beschrieben in « The Antibody Molecule », Academic Press New York, 1975, 322-326. Zur Herstellung der reduzierenden Bedingungen werden in der Regel SHgruppenhaltige Verbindungen zugesetzt, beispielsweise Cystein, Mercaptoäthanol und ähnliche. Die Inkubation mit dem proteolytischen Enzym wird abgestoppt, wenn die Spaltung vervollständigt ist, zweckmäßig durch Zusatz eines der bekannten Inaktivierungsmittel. Wie oben bereits erwähnt, wird im Rahmen der Erfindung Jodacetamid bevorzugt. Die bei der Spaltung entstandenen freien SH-Gruppen der FAB-Fragmente werden durch dieses Abstoppmittel alkyliert. Auf diese Weise behandelte FAB-Fragmente eignen sich im Rahmen der Erfindung besonders gut, insbesondere, wenn auch noch restliche Antikörper und $F_c$-Fragmente entfernt werden. Die Hemmung der CK-MB erfolgt dann völlig ohne Auftreten von Präzipitaten und beträgt praktisch genau 50%. Jedoch lassen sich im Rahmen der Erfindung auch solche FAB-Fragmente verwenden, die nicht alkyliert sind oder von Begleitstoffen nicht gereinigt sind, wenn dadurch auch ein etwas größerer Fehler bei der Bestimmung erhalten wird, der jedoch immer noch im Rahmen des gut Brauchbaren liegt, d.h. zu 50 % ± 3 % das Hybridenzym hemmt.

Die Bestimmung der Untereinheit B im Hybridenzym CK-MB nach Zusatz der monovalenten Antikörper erfolgt dann nach den für die CK-Bestimmung generell bekannten Methoden, die von Creatin oder von Creatinphosphat als Substrat ausgehen. Geeignete Verfahren sind beispielsweise beschrieben in « Methoden der enzymatischen Analyse », von H. U. Bergmeyer, Verlag Chemie, Band 1, 3. Auflage, Seite 813-825. Ein aufgrund seiner hohen Empfindlichkeit besonders geeignetes Verfahren ist in der deutschen Patentanmeldung P 29 08 054.4 (eingereicht am 2. März 1979 unter der internen Nr. 2292 von der gleichen Anmelderin) beschrieben. Diese Methode wendet die Luciferin-Luciferase-Reaktion an und mißt die emittierte Lichtmenge kinetisch.

Das erfindungsgemäße Verfahren ermöglicht den Einsatz von Antiseren gegen CK-MM zur quantitativen Bestimmung von CK-MB, die nach irgendeiner der in der Literatur beschriebenen Immunisierungsmethoden gewonnen wurden und bisher wegen zu hoher CK-MB-Hemmung nicht für einen diagnostisch zuverlässigen CK-MB-Test im Serum anwendbar waren. Auch Antiseren, die neben der Hemmung Präzipitation verursachen, können erfindungsgemäß ausgenützt werden. Die selbst bei ausgefeilten Immunisierungsmethoden in sehr beträchtlichem Ausmaß anfallenden Antiseren mit zu hoher CK-MB-Hemmung brauchen nicht mehr ausgesondert zu werden, es entfällt die aufwendige Analytik für die Differenzierung zwischen brauchbaren und nicht brauchbaren Antiseren. Letzteres ist von besonderer Bedeutung, da die Austestung der CK-MB-Hemmung eines Antiserums nur an CK-MB-Präparaten aus frischem Serum von Herzinfarkt-Patienten möglich ist. Der Wegfall des Hemmtests führt daher zu einer erheblichen Vereinfachung.

Die folgenden Beispiele erläutern die Erfindung weiter.

Beispiel 1

a) Herstellung der IgG-Fraktion

Serum von Schafen, die nach dem in « Immunologische Arbeitsmethoden », loc. cit., beschriebenen Schema mit humaner CK-MM immunisiert waren, wird mit kristallinem Ammoniumsulfat bei 0 bis 4 °C und pH 7 ad 1,8 M versetzt. Nach Zentrifugation wird der Niederschlag in 0,1 M TRIS/0,15 M NaCl-Puffer, pH 8, gelöst und ausgiebig gegen 10 mM Phosphatpuffer, pH 7,1, dialysiert. Das nochmals durch Zentrifugation geklärte Dialysat wird auf eine Säule aufgetragen, die mit DEAE-Cellulose gefüllt ist. Der proteinhaltige Durchlauf und das Eluat, das mit 15 mM Phosphat/10 mM NaCl-Puffer, pH 7,1, erhalten wird, werden gereinigt und enthalten mehr als 95 % reines IgG.

b) Papain-Spaltung

IgG wird nach dem in Immunochem. 4 (1967), 369 beschriebenen Verfahren unter reduzierenden Bedingungen (10 mM Cystein) mit 1,5 % Papain (Gew.-%, bezogen auf Menge IgG-Protein) bei pH 7,5 über 5 Stunden bei 37 °C inkubiert. Am Ende der Inkubationsphase wird die Enzymeinwirkung durch einen Überschuß an Jodacetamid gestoppt und zur Alkylierung von freien SH-Gruppen bei pH 7,5 zwei Stunden bei Raumtemperatur inkubiert. Das stabilisierte Spaltgemisch enthält FAB-Fragmente, $F_c$-Fragmente und < 10 % ungespaltenes IgG. Die Immunreaktivitätsausbeute beträgt mehr als 75 %.

c) Reinigung der FAB-Fragmente

Nach Konzentration auf 30 mg/ml durch Ultrafiltration wird das Spaltgemisch durch Gel-Chromatographie (The LKB Instrument Journal 24, (1977) 10) aufgetrennt. FAB-Fragmente eluieren als letzter Proteinpeak gut getrennt von IgG und $F_c$-Fragmenten. Die FAB-Fraktion kann nach Konzentrierung durch Ultrafiltration direkt zur Hemmung von CK-MM oder CK-MB eingesetzt werden. Die Hemmung von CK-MB beträgt im Rahmen der Fehlergrenze 50 %. Die Hemmung von CK-MM liegt bei 99 bis 100 %.

Beispiel 2

a) Herstellung der γ-Globulin-Fraktion aus Schafplasma

Citratplasma von Schafen, die 4 bis 6 Monate mit CK-MM immunisiert wurden, wird ad 25 mM Calciumchlorid versetzt und ca. 2 Stunden bei Raumtemperatur zur Gerinnung angesetzt. Nach mechanischer Zerteilung des Gerinnsels wird mit 1 Volumen physiologischer NaCl-Lösung verdünnt und zur Lipoproteinadsorption mit 2 % Silicat-Flockungsmittel (Aerosil) versetzt.

Durch Zentrifugation wird der klare Überstand gewonnen. Aus dem Überstand wird bei 0 bis 4 °C und pH 7 mit 1,8 M Ammoniumsulfat die γ-Globulin-Fraktion gefällt und durch Zentrifugation gesammelt. Durch Dialyse gegen 50 mM TRIS/0,1 M NaCl-Puffer, pH 7,5, und Konzentration durch Ultrafiltration erhält man ein für die enzymatische Spaltung geeignetes Antikörper-Präparat.

b) Papain-Spaltung

Es wird wie in Beispiel 1, b) beschrieben, verfahren, jedoch wird für die γ-Globulin-Fraktion geringfügig modifiziert. 1,5 % Papain werden bezogen aud g Protein der γ-Globulin-Fraktion und die Inkubationszeit beträgt 20 Stunden bei 25 °C. Sonstige Bedingungen sind gleich.

c) Reinigung der FAB-Fraktion

Das alkylierte Spaltgemisch wird dialysiert gegen physiologische Kochsalzlösung, dann mit 25 mM Zinksulfat versetzt (Immunochem. 4, (1977) 99). Dadurch werden Reste ungespaltenen IgGs und $F_c$-Fragmente ausgefällt und durch Zentrifugation abgetrennt. Die verbleibenden FAB-Fragmente sind nach Dialyse und Konzentration fertig zur Verwendung im CK-MB-Test. Die Ausbeute beträgt mehr als 80 % der Hemmaktivität für CK-MM-Isoenzym, bezogen auf das Ausgangsplasma. CK-MB wird unabhängig vom Hemmwert für die ungespaltenen Antikörper zu 50 % gehemmt. Die mit verschiedenen Seren erhaltenen Ergebnisse zeigt die nachstehende Tabelle.

TABELLE

| Tier-Nr. | CK-MM (600-1 000 U/l) | | CK-MB (200-400 U/l) | |
|---|---|---|---|---|
| | % Hemmung der Enzymaktivität | | | |
| | Ausgangsserum (Plasma) | FAB-Fragmente | Ausgangsserum | FAB-Fragmente |
| 1 | 99,5 | 99,5 | 62 | 52 |
| 2 | 99,6 | 99,7 | 56 | 53 |
| 3 | 99,3 | 99,1 | 64 | 49 |
| 4 | 98,5 | 99,3 | 68 | 52 |

Ansprüche

1. Verfahren zur Bestimmung der Creatinkinase-MB im Serum durch immunologische Ausschaltung der Untereinheit M und Messung der Untereinheit B nach bekannten Methoden zur Bestimmung der Creatinkinase, dadurch gekennzeichnet, daß man zur Ausschaltung der Untereinheit M im Reaktionsansatz aus Antikörpern gegen die Untereinheit M durch proteolytische Spaltung unter reduzierenden Bedingungen gewonnene monovalente Fragmente zusetzt.

2. Verfahren nach Anspruch 1, dadurch gekennzeichnet, daß man monovalente Fragmente verwendet, die aus Antikörpern gewonnen werden, welche das Hybridenzym CK-MB zu mehr als 55 % und das Isoenzym CK-MM vollständig hemmen.

3. Verfahren nach Anspruch 2, dadurch gekennzeichnet, daß man monovalente Fragmente von Antikörpern, welche das Hybridenzym CK-MB zu 60 bis 90 % hemmen, verwendet.

4. Verfahren nach einem der vorhergehenden Ansprüche, dadurch gekennzeichnet, daß man monovalente Fragmente verwendet, aus welchen die restlichen vollständigen Antikörper und die kristallisierbaren Fragmente (F$_c$) abgetrennt sind.

5. Verfahren nach einem der vorhergehenden Ansprüche, dadurch gekennzeichnet, daß man monovalente Fragmente zusetzt, deren SH-Gruppen alkyliert sind.

6. Verfahren nach Anspruch 5, dadurch gekennzeichnet, daß man mit Jodacetamid oder Jodacetat alkylierte monovalente Fragmente zusetzt.

7. Verfahren nach einem der vorhergehenden Ansprüche, dadurch gekennzeichnet, daß man die Untereinheit B mit Hilfe der Luciferin-Luciferase-Reaktion bestimmt und die emittierte Lichtmenge kinetisch mißt.

8. Reagenz zur Bestimmung der Creatinkinase-MB, enthaltend ein System zur Bestimmung der Creatinkinase-MB und ein Mittel zur immunologischen Ausschaltung der Untereinheit M der Creatinkinase, dadurch gekennzeichnet, daß das Mittel zur immunologischen Auschaltung aus monovalenten Fragmenten von Antikörpern gegen Creatinkinase-MM besteht.

## Claims

1. Process for the determination of creatine kinase-MB in serum by immunological elimination of subunit M and measurement of subunit B by methods known for the determination of creatine kinase, characterised in that, for the elimination of subunit M in the reaction batch, one adds monovalent fragments obtained from antibodies against subunit M by proteolytic splitting under reducing conditions.

2. Process according to Claim 1, characterised in that one uses monovalent fragments which are obtained from antibodies which inhibit the hybrid enzyme CK-MB by more than 55 % and the isoenzyme CK-MM completely.

3. Process according to Claim 2, characterised in that one uses monovalent fragments from antibodies which inhibit the hybrid enzyme CK-MB by 60 to 90 %.

4. Process according to one of the preceding Claims, characterised in that one uses monovalent fragments from which the residual complete antibodies and the crystallisable fragments (F$_c$) are separated.

5. Process according to one of the preceding Claims, characterised in that one adds monovalent fragments, the SH groups of which are alkylated.

6. Process according to Claim 5, characterised in that one adds monovalent fragments alkylated with iodoacetamide or iodoacetate.

7. Process according to one of the preceding Claims, caracterised in that one determines the subunit B with the help of the luciferin-luciferase reaction and measures the emitted amount of light kinetically.

8. Reagent for the determination of creatine kinase-MB, containing a system for the determination of creatine kinase-MB and an agent for the immunological elimination of the subunit M of the creatine kinase, characterised in that the agent for the immunological elimination consists of monovalent fragments from antibodies against creatine kinase-MM.

## Revendications

1. Procédé pour le dosage de la créatinekinase-MB dans le sérum par élimination immunologique de la sous-unité M et mesure de la sous-unité B selon des méthodes connues pour le dosage de la créatinekinase, caractérisé en ce que, pour éliminer la sous-unité M, on ajoute dans le milieu réactionnel des fragments monovalents obtenus à partir d'anticorps contre la sous-unité M par clivage protéolytique dans des conditions réductrices.

2. Procédé selon la revendication 1, caractérisé en ce qu'on utilise des fragments monovalents qui sont obtenus à partir d'anticorps qui inhibent l'enzyme hydride CK-MB à plus de 55 % et l'isoenzyme CK-MM complètement.

3. Procédé selon la revendication 2, caractérisé en ce qu'on utilise des fragments monovalents d'anticorps qui inhibent à 60 à 90 % l'enzyme hydride CK-MB.

4. Procédé selon l'une des revendications précédentes, caractérisé en ce qu'on utilise des fragments monovalents à partir desquels les anticorps complets restants et les fragments cristallisables (F$_c$) sont séparés.

5. Procédé selon l'une des revendications précédentes, caractérisé en ce qu'on ajoute des fragments monovalents dont les groupes SH sont alcoylés.

6. Procédé selon la revendication 5, caractérisé en ce qu'on ajoute des fragments monovalents alcoylés par l'iodoacétamide ou l'iodoacétate.

7. Procédé selon l'une des revendications précédentes, caractérisé en ce qu'on dose la sous-unité B à l'aide de la réaction luciférine-luciférase et qu'on mesure cinétiquement la quantité de lumière émise.

8. Réactif pour le dosage de la créatinekinase-MB, comprenant un système pour le dosage de la créatinekinase-MB et un agent pour l'élimination immunologique de la sous-unité M de la créatinekinase, caractérisé en ce que l'agent pour l'élimination immunologique est constitué de fragments monovalents d'antico.ps contre la créatinekinase-MM.